# EUROPEAN PATENT APPLICATION

(11) **EP 4 418 075 A1**
(43) Date of publication of application: **21.08.2024**
(21) Application number: 23157349.4
(22) Date of filing: 17.02.2023
(51) Int. Cl.: G06F 3/01, G06F 1/16, G02B 27/01, A61B 5/256

(54) **HUMAN-MACHINE INTERFACE, KIT-OF-PARTS AND METHODS FOR ADJUSTING**

(71) Applicant: Chaudhary, Ujwal, 72074 Tübingen (DE)
(72) Inventor: Chaudhary, Ujwal, 72074 Tübingen (DE)
(74) Representative: Ullrich & Naumann PartG mbB

(57) **Abstract**

The present invention relates to a Human-Machine Interface (HMI), comprising a wearable unit (1), preferably a head-worn unit, one or more sensors (2a, 2b, 2c, 2d, 2e, 2f, 2g, 2h, 2i, 2j, 2k, 2l, 2m, 2n, 2o, 2p, 2q, 2r, 2s, 2t) for collecting user data, wherein at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t) is connectable to the wearable unit (1), and a data processing system (5) for acquiring and processing said user data, wherein the wearable unit (1) is configured to be connected to at least two different hardware modules, such that the wearable unit is customizable for an individual user.

The invention further relates to a kit-of-parts for the Human-Machine Interface (HMI) and to methods for adjusting the Human-Machine Interface (HMI).

## Description

### FIELD OF THE INVENTION

The present invention relates to a Human-Machine Interface. Further, the present invention relates to a kit-of-parts for the Human-Machine Interface and to methods for adjusting the Human-Machine Interface. Embodiments of the invention have been particularly developed as assistive devices that facilitate communication. For example, embodiments of the invention aim at assisting users to communicate, who suffer from motor neuron disorders, brain trauma or other neurological conditions. The invention will be described hereinafter with reference to this application. However, it will be appreciated that the invention is not limited to this particular field of use.

### BACKGROUND OF THE INVENTION

Any discussion of the background art throughout the specification should in no way be considered as an admission that such art is widely known or forms part of common general knowledge in the field.

For the purpose of the present specification, the "Human-Machine Interface" is to be understood in the broadest possible sense of an interface between a human and a machine, in particular including embodiments in which the machine is or comprises a computer. These types of Human-Machine Interfaces - "HMis" - are sometimes referred to as Human-Computer Interfaces "HCls". However, HMIs or HCls as referred to in the present disclosure are to be distinguished from known head-mounted displays - "HMDs" - such as virtual reality headsets used for recreational or entertainment purposes.

Communication is the process of expressing and sharing feelings, thoughts and intentions with one another by verbal and various non-verbal means. Known HMls can be used to establish communication between a user of the HMI and the respective machine. Ultimately, the user may communicate to other people via the HMI and the machine.

Known HMls are able to acquire and use physiological signals or bio-signals of a user in order to drive external machines or devices. For example, known HMIs may include a head-worn unit and sensors for collecting electroencephalography - "EEG" - user data from the user. Based on this user data, the HMI may try to identify what it is that a user wants to express. Successful interaction with an HMI may require feedback to the user and/or training with the HMI. For example, the user may be confronted with simple YES/NO-Questions. The user's brain activity corresponding to YES-Answers may be correlated with a pattern in the EEG-user data, and the user's brain activity corresponding to NO-Answers may be correlated with a different pattern in the EEG-user data. Afterwards, each brain activity relating to YES/NO-Questions can be matched to either a YES-Answer or a NO-Answer given by the user by matching the data pattern found in the user data relating to that specific brain activity with the known data patterns relating to either YES or NO. Such feedback and/or training may be expanded to identify for example numbers, letters in the alphabet, syllables and/or characters, and establish complex communication between the user and the HMI and - ultimately - between the user and other people via the HMI.

However, the design of known HMIs is not ideal. Known HMIs regularly comprise bulky units connected to or worn on a user's head, with a vast amount of structurally identical sensors distributed over the bulky unit. Known HMIs often have an obtrusive appearance and known HMIs are regularly not suitable for use outside of a laboratory environment.

Further, known HMIs typically do not allow for flexible application over a broad variety of use cases, but are rather designed to use a specific type of sensor in order to acquire a specific type of signal as user data from all kinds of possible users in the same way.

It is an object of the present invention to overcome or ameliorate at least one of the disadvantages of the prior art, or to provide a useful alternative. In particular, it is an object of the present invention to improve and further develop an HMI, such as to allow for flexible application over a broad variety of use cases and areas of application. Another object of the invention is to provide an HMI that is more adaptable to the needs of an individual user.

### SUMMARY OF THE INVENTION

In a first aspect, the present invention provides a Human-Machine Interface, comprising a wearable unit, preferably a head-worn unit, one or more sensors for collecting user data, wherein at least one of the one or more sensors is connectable to the wearable unit, and a data processing system for acquiring and processing said user data, wherein the wearable unit is configured to be connected to at least two different hardware modules, such that the wearable unit is customizable for an individual user.

In a second aspect, the present invention provides a Kit-of-parts for the Human-Machine Interface according to the first aspect, comprising a wearable unit, preferably a head-worn unit, at least two different hardware modules, such that the wearable unit is customizable for an individual user, wherein the at least two different hardware modules are connectable to the wearable unit, and wherein the at least two different hardware modules can be different types of sensors for collecting user data and/or different types of extensions of the wearable unit for positioning sensors for collecting user data, and optionally a data processing system for acquiring and processing said user data.

In a third aspect, the present provides a method for adjusting the Human-Machine Interface according to the first aspect, comprising the steps of
recording data collected by the one or more sensors over time,
monitoring data patterns associated with respective identified individual user signals for a relevant change, and
upon detecting a relevant change in a data pattern associated with a respective individual user signal, taking one of the following adjustment measures according to the requirements of the individual user:
   a) adapting the sensitivity and/or configuration and/or localization of at least one of the one or more sensors;
   b) exchanging at least one of the at least two different hardware modules, preferably exchanging one of the one or more sensors;
   c) connecting at least one of the at least two different hardware modules, preferably adding an extension of the wearable unit for positioning at least one of the one or more sensors for acquiring data from an additional region of an individual user's head and/or body; and/or
   d) disconnecting at least one of the at least two different hardware modules.

In a fourth aspect, the present invention provides a method for adjusting the Human-Machine Interface according to the first aspect, comprising the steps of
recording data collected by the one or more sensors over time,
acquiring additional signals from sensors, and
depending on the additional signal, taking one of the following adjustment measures according to the requirements of the individual user:
   a) adapting the sensitivity and/or configuration and/or localization of at least one of the one or more sensors;
   b) exchanging at least one of the at least two different hardware modules, preferably exchanging one of the one or more sensors;
   c) adding at least one of the at least two different hardware modules, preferably adding an extension of the wearable unit for positioning at least one of the one or more sensors for acquiring data from an additional region of an individual user's head and/or body; and/or
   d) disconnecting at least one of the at least two different hardware modules.

### DETAILED DESCRIPTION OF THE INVENTION

In accordance with the invention, the aforementioned objects are accomplished by a Human-Machine-Interface comprising the features of claim 1. According to claim 1, a Human-Machine Interface comprises a wearable unit, preferably a head-worn unit, one or more sensors for collecting user data, wherein at least one of the one or more sensors is connectable to the wearable unit, and a data processing system for acquiring and processing said user data.

The Human-Machine Interface according to claim 1 is characterized in that the wearable unit is configured to be connected to at least two different hardware modules, such that the wearable unit is customizable for an individual user.

The wearable unit can preferably be head-worn. The term "wearable unit" may also extend to units that may be placed, attached or worn on the individual user's body, for example to position at least one of the one or more sensors on or near the individual user's body. Such a configuration can advantageously be employed for denoising of signals acquired by at least one other of the one or more sensors.

Where the wearable unit is a head-worn unit, it can for example be realized as spectacles, as a hat, as earpieces with or without a connecting bow, as a noseplug and/or as other known types of head-worn units.

The data processing system for acquiring and processing said user data may be any type of data processing system suitable to interpret signals. Such interpretation may include recognition and classification of a signal that generates data, in particular user data. The signals may be signals of the individual user, for example bio-signals such as eye movement or gaze fixation, muscle contractions, brain activity or other types of bio-signals. The bio-signals may be electrical signals that are collectable by at least one of the one or more sensors. In addition to interpreting user data, at least one sensor may also be configured to collect data from signals that are independent from the individual user, for example signals from the environment of the HMI, such as ambient light, temperature, air pressure, humidity and/or other types of environment signals.

In an inventive manner, it has been recognized that there is no wearable HMI system that is adaptable to each individual user as well as usable outside of a laboratory environment.

The wearable unit may be configured to be connected to at least two different hardware modules, for example by providing hardware interfaces such as ports, connectors, plugs, cables and/or other known types of hardware interfaces. Additionally or alternatively, the wearable unit may be configured to be connected to at least two different hardware modules by way of a wireless connection, such as Wi-Fi, Near Field Communication "NFC", Wireless Personal Area Network technology such as Bluetooth, or other appropriate technologies.

By way of configuring the wearable unit to be connected to at least two different hardware modules, the wearable unit becomes customizable for each individual user. While known HMIs are typically configured to be connected to merely one type of hardware module - routinely via many identical sensors of the same type - the HMI according to the present invention is configured to be connected to at least two different types of hardware modules. Accordingly, while one individual user may require a first type of hardware module to best use the HMI according to their individual needs, another individual user may require a second type of hardware module that is different from the first type of hardware module. The at least two different hardware modules create a combinatorial yet modular setup for the HMI, so that hardware modules may be added, removed, interchanged, and/or exchanged. Since at least two different hardware modules are connectable, the modular HMI can be adapted to best meet the needs and preferences of an individual user.

Accordingly, the proposed HMI allows for flexible application over a broad variety of use cases such as use outside of a laboratory environment or adapting the HMI to an individual user. Also, the proposed HMI is adaptable to the needs of the individual user and therefore personalizable.

Preferably, one of the one or more sensors may be realized as one of the at least two different hardware modules. The wearable unit may comprise sensors that are fixedly connected to the wearable unit and are not exchangeable. Additionally, the wearable unit may comprise interfaces for connecting and/or exchanging different sensors and/or different sensor types in order to customize the HMI for the individual user. For example, the at least two different hardware modules may be two different sensors and/or two different sensor types. In other embodiments, one of the two different hardware modules may be a sensor, and the other hardware module may be realized as a different type of hardware module. At least one of the at least two different hardware modules may be an HMI hardware module such as one of the one or more sensors.

One of the sensors realized as one of the hardware modules of the HMI may include an implant to collect a bio-signal of the individual user invasively. For example, one of the sensors realized as one of the hardware modules may be realized as a headstage, which is a component that comprises an implant and protrudes out of the skull of the individual user. Such a sensor can be used to record user data in the form of a bio-signal invasively collected from the brain of the individual user. In addition to the data processing system of the HMI, the headstage may be configured to not only collect user data, but to also analyze and interpret the invasively collected bio-signal as well as transmit the recorded, analyzed and interpreted invasively collected bio-signal to other external devices.

One of the hardware modules of the HMI may have an additional functionality to interface - physically and/or wirelessly - with an additional implanted sensor to collect a bio-signal of the individual user. In that case, one or more attachable/detachable unit of the hardware will have the capability to not only collect user's data, but to also analyze and interpret the invasively collected bio-signal as well as transmit the recorded, analyzed and interpreted invasively collected bio-signal to other external devices.

Preferably, the at least one of the one or more sensors may include an electrode for collecting electrooculography "EOG", electromyography "EMG", and/or electroencephalography "EEG" user data. One or more of such electrodes may be fixedly connected to the wearable unit and may not be exchangeable. Alternatively or additionally, one or more of such electrodes may be realized as one of the at least two different hardware modules such as to be customizable for an individual user.

Further, preferably, the data processing system may be configured to process user data from at least two modalities comprising electrooculography "EOG", electromyography "EMG", and electroencephalography "EEG" in order to interpret an individual user's signal. When user data is acquired by at least two modalities comprising EOG, EMG and EEG, the interpretation of a signal of the individual user can be improved. Interpretation of an individual user's signal can be based on two or more different sets of user data collected from at least two of the modalities. For example, user data collected by one of the modalities can be applied to verify an interpretation of user data that is based on user data collected by another one of the modalities.

Additionally or alternatively, the one or more sensors may include a sensor or a set of sensors to detect single unit activity "SUA" and/or multiunit activity "MUA", local field potential "LFP", functional ultrasound "fUS" and/or functional near infrared spectroscopy "fNIRS". Further sensors may include an accelerometer, a gyroscope, sensors for light, sensors for sound and/or sensors associated with a bone transduction speaker. Such sensors may collect user data - for example from at least one bio signal - and/or data from signals that are independent from the individual user.

Preferably, the sensors include at least two different kinds of sensors for collecting time series signals and/or frequency series signals. For example, the at least two different kinds of sensors may be two different kinds of electrodes for collecting the same and/or different bio signals such as the modalities named above. The user data collected from such time series signals and/or frequency series signals can be analyzed and interpreted in a favorable way in order to improve communication.

Further preferably, the signals may include an invasively and/or non-invasively collected bio-signal and/or a blood oxygenation level-dependent signal. Such signals may be an intended input actively generated by the individual user and/or a signal passively generated by the individual user by which a state of the individual user may be detected. User data from such signals - alone or in combination with data from one or more of the signals described above - allows for advanced processing and interpretation, for example by the data processing system of the HMI.

In one possible field of application of the HMI, the individual user may be a patient suffering from motor neuron disorders, brain trauma or other neurological conditions causing impaired movement. While communication skills among healthy individuals appear automatic, communication can pose severe challenges to such patients. When a patient can no longer communicate verbally, they may use different assistive and augmentative communication devices "AACs" that rely on non-verbal signals such as finger movement and/or gaze fixation for communication. Known AACs are bulky and do not function once an individual loses the ability to - for example - move their finger and/or fixate their gaze. To help these users to communicate, the described HMI may be used as an AAC to provide a reliable external pathway to bypass the disrupted motor pathway, such that interaction between the brain of the patient and another person or an external device/instrument becomes possible. The described HMI can be used to establish communication with paralyzed patients.

Preferably, the data processing system can be configured to provide the individual user with information, wherein said information is provided in a format perceivable by the individual user. This way, the individual user may be guided and/or trained on how to interact with the HMI. For example, the data processing system may provide the individual user with feedback on a signal that he has purposely generated as input in order to communicate. The information may be provided by way of video and/or text via a display and/or via other suitable devices.

Preferably, the data processing system can be configured to provide the individual user with information in a non-visual format. For example, the information may be provided by way of audio or tactile signals for haptic perception. Providing non-visual information is particularly advantageous with individual users that are patients suffering from blindness or vision impairment and are not capable of perceiving visual information such as video and/or text. Such vision impairment is not uncommon among patients suffering from motor neuron disorders, brain trauma or other neurological conditions. In particular, in preferred embodiments, the HMI does not comprise a display device.

Preferably, the wearable unit may comprise speaker and/or display devices. Such devices may be employed to provide the individual user with information, in a visual or non-visual format such as audio. Such information may include feedback of the HMI provided to the user for communication purposes. Additionally or alternatively, the HMI may comprise speaker and/or display devices for communication with the outside world, in particular to express and share feelings, thoughts and intentions with other people. Speakers for communication with the individual user may be realized as earpieces associated with the wearable unit, but may additionally or alternatively include a separate speaker system. The wearable unit may comprise a suitable interface using via ports, connectors, plugs, cables and/or other known types of hardware interfaces. The connection to the separate speaker system may also be established by way of a wireless connection, wireless data transmission such as such as Wi-Fi, Near Field Communication "NFC", Wireless Personal Area Network technology such as Bluetooth, or other appropriate technologies.

Preferably, the data processing system comprises a data acquisition unit and/or a data analysis unit and/or a data interpretation unit.

The data acquisition unit "DAQ" may be configured to record data from the sensors described above, including but not limited to the EOG, EMG, EEG and also in ear EEG from a region of interest, typically a head or body region of the individual user. Additionally or alternatively, the DAQ may be configured to acquire additional data from the sensors described above, including user data from movement signals and/or data relating to heart rate variability "HRV" of the individual user using an integrated accelerometer, gyroscope and other types of sensors.

The data analysis unit may be configured to analyze data acquired by the DAQ. For example, the DAQ may perform one or more of the following tasks: Normalization, artifact rejection, baseline correction, filtering, smoothing, movement correction, movement compensation, dimensionality reduction and/or other suitable data analyzation tasks.

The data interpretation unit may be configured to interpret the data. The main task of the data interpretation unit will be to interpret an individual user's signal based on the respective user data collected by the sensors and acquired by the DAQ in order to identify an input the user has purposely imposed on the HMI. As described above, the DAQ may also identify input signals passively generated by the individual user. If there is no detectable input, then the device may be configured to go into standby mode. In another example, the HMI may be configured to go into standby mode depending on the state of the individual user. For instance, when monitoring and/or supervising the state of the individual user, the data processing system may detect that the individual user is sleeping, which may also cause going into standby mode. When in standby mode, the DAQ may be configured to acquire data in intervals in order to identify input signals passively and/or actively generated by the individual user. Such an interval may be one, two to five, five to ten and/or 15 or more minutes, depending on the specific field of application and on the specific needs of the individual user. The standby mode can be exited, once the data processing system identifies detectable input again or, for example, once the acquired data suggests that the individual user's state is changing or has changed.

The data interpretation unit may be realized as an intelligent data processing and communication unit. The data interpretation unit may be configured to processes all the data acquired by the DAQ including but not limited to the EOG, EMG, EEG, and ear EEG data using an artificial intelligence algorithm "Al" and/or statistical algorithms to recognize and classify a signal according to the intent of the user, for example YES/NO-Answers and/or selections of an option. The data interpretation unit can be connected to the DAQ, the data analysis unit and/or the wearable unit. User data and/or other data can transferred from DAQ to the data interpretation unit. The data interpretation unit may send a command to provide the individual user with information as described, for example by playing questions, instructions and/or commands to the individual user.

Once the command from the data interpretation unit is sent, the respective information is provided to the individual user in a format perceivable by the individual user, in order to make individual user aware of options within the HMI communication system. For instance, these options can be but are not limited to questions with YES/NO-answers, predefined words, phrases and sentences, and alphabets, colors, and numbers. The information may include instructions to guide the individual user to communicate his wishes and desires. For example, Chaudhary et al. 2022 (Nature communications 13.1 (2022): 1236; the disclosure of which is incorporated by reference), describe how auditory neurofeedback training can be utilized to enable the use of a spelling interface for communication, even if the user has lost all other means of communication. The data interpretation unit may run Al and/or statistical algorithms online and/or offline and/or an online data stream to enable communication with the user. Thus, the information - for example audio instructions - offers personalized depth by considering each individual user's preferred communication choices, preferences, and abilities. Jaramillo-Gonzalez et al. 2021 (Scientific data 8.1 (2021): 8; the disclosure of which is incorporated by reference) and Tonin et al. 2020 (Scientific reports 10.1 (2020): 8452; the disclosure of which is incorporated by reference) describe the general concepts of training systems and data interpretation methods and algorithms also suitable for use in the context of the present invention.

Known HMIs have been used by healthy individual users and patients in a laboratory environment. The HMI disclosed herein may be fully customizable and wearable for communication that is user-friendly and easy to use even outside such a laboratory environment.

In embodiments of the HMI, the data acquisition unit and/or a data analysis unit and/or the data interpretation unit may be realized in separate hardware devices. The data acquisition unit may be realized in a first separate hardware device, while the data analysis unit and the data interpretation unit may be realized in a second separate hardware device. Alternatively, the data analysis unit and the data interpretation unit may also be realized in two separate hardware devices. For example, the DAQ may be a separate device worn on the body of the individual user.

The DAQ may be connectable to the wearable unit and/or to the one or more sensors via ports, connectors, plugs, cables and/or other known types of hardware interfaces. Additionally or alternatively, the DAQ may be connectable to the wearable unit and/or to the one or more sensors by way of a wireless connection, wireless data transmission such as such as Wi-Fi, Near Field Communication "NFC", Wireless Personal Area Network technology such as Bluetooth, or other appropriate technologies.

The data analysis unit and/or the data interpretation unit may be connectable to the DAQ, for example via ports, connectors, plugs, cables and/or other known types of hardware interfaces. Additionally or alternatively, the data analysis unit and/or the data interpretation unit may be connectable to the DAQ by way of a wireless connection, wireless data transmission such as such as Wi-Fi, Near Field Communication "NFC", Wireless Personal Area Network technology such as Bluetooth, or other appropriate technologies. The data analysis unit and/or the data interpretation unit may be realized as one or two separate mobile devices such as a computer, tablet or smartphone.

In preferable embodiments of the HMI, the data acquisition unit and/or the data analysis unit and/or the data interpretation unit may be integrated into a single hardware device such as a computer, tablet or smartphone. The data acquisition unit and/or the data analysis unit and/or the data interpretation unit may be connectable to the wearable unit and/or to the one or more sensors via ports, connectors, plugs, cables and/or other known types of hardware interfaces. Additionally or alternatively, the data acquisition unit and/or the data analysis unit and/or the data interpretation unit may be connectable to the wearable unit and/or to the one or more sensors by way of a wireless connection, wireless data transmission such as such as Wi-Fi, Near Field Communication "NFC", Wireless Personal Area Network technology such as Bluetooth, or other appropriate technologies.

Preferably, and regardless of integration of the data acquisition unit and/or the data analysis unit and/or the data interpretation unit into one or several hardware devices, the data processing system in its entirety may be stationary, for example for use in a laboratory environment.

More preferably, however, the data processing system in its entirety may be portable and/or movable, so that the individual user may move and/or be moved to a different location while using the HMI, for example realized as one or more mobile devices such as a computer, tablet or smartphone.

Further more preferably, the data processing system in its entirety may be wearable by the individual user, so that the individual user may wear the entire HMI in everyday life. For example, the data processing system or its component units may be realized as a body-worn mobile device and/or integrated into the wearable unit, which may preferably be a head-worn unit.

In a most preferred embodiment, the data processing system or its component units may be realized in a transparent manner, meaning they can be worn by the individual user in an unobtrusive way in order to achieve minimum restriction or limitation of the individual user in everyday life.

The data processing system in its entirety may be integrated into a mobile device such as a computer, tablet or smartphone. One, two or all three of the component units may be realized as an app, preferably connectable to the wearable unit and/or to the one or more sensors by way of a wireless connection, wireless data transmission such as such as Wi-Fi, Near Field Communication "NFC", Wireless Personal Area Network technology such as Bluetooth, or other appropriate technologies.

Preferably, one of the at least two different hardware modules may be implemented as an extension of the wearable unit, wherein the extension can comprise at least one of the one or more sensors for collecting user data from at least one additional region of the individual user's head and/or body. In addition to the region of interest, where user data can be collected via the at least one sensor associated with the wearable unit, such an extension enables the HMI to collect user data from at least one additional region of the users head and/or body not reachable by the wearable unit without the extension. Just like the wearable unit, the extension may also comprise at least one sensor configured to collect data from signals that are independent from the individual user, for example signals from the environment of the HMI, such as example ambient light, temperature, air pressure, humidity and/or other types of environment signals. The wearable unit may comprise interfaces for connecting and/or exchanging different extensions and/or different types of extensions in order to customize the HMI for the individual user. One or more of the sensors on the extension may in turn be realized as connectable hardware modules, for example to be attachable/detachable to/from the extension.

Preferably, the wearable unit comprises a spectacle-like frame, preferably including rims, hinges and/or temples. Further, preferably, the wearable unit comprises two rims, two hinges and two temples so that the individual user may wear the wearable unit just like a pair of glasses. In one exemplary embodiment, the rims may be realized as hollow rims and/or the hinges may be realized as hollow hinges. One or more of the extensions of the wearable unit may be realized as a matching temple or bracket to match the spectacle-like frame.

For example, a spectacle rim may extend to touch the individual user's skin above the eyebrows and around the lateral orbital rim of the eye. The spectacle's rims, hinges, and temples may have integrated sensors, such as electrodes touching the skin above the eyebrows and around the orbital rim of the eye. One or more of these sensors may also - as described - be realized as one of the at least two different hardware modules. The sensors may record any change in potential associated with the ocular movement of the user. The connection of the sensors integrated into the spectacle's rim, hinges and temples can pass from inside the hollow rim through the hollow hinges and temples in order to connect to the DAQ. The wearable unit may comprise slots to attach one or more of the extensions in order to extend the area coverage and functionality of the HCI system to additional regions.

Further, preferably, the wearable unit may comprise speaker devices realized as at least one earpiece. The earpiece may comprise a sound outlet attached at an endpiece - in other words at an end of a temple - of the spectacle-like frame.

An earpiece attached to the spectacle-like frame may additionally comprise one or more sensors able to record an EEG signal from inside the ear of the individual user. The sensor on the earpiece may be realized as a ring electrode surrounding the earpiece or as an electrode of any other suitable configuration. Further, the data processing system may have an interface with the earpiece. Accordingly, the earpiece can also be adapted to play questions, instructions, and commands directly into the individual user's ear(s) as described above. The earpiece may be realized as an added functionality to the functionality to play the questions, instructions, and commands directly from the data processing system. Additionally and/or alternatively, the speaker devices may include one or more bone transduction speakers.

Preferably, the wearable unit may comprises a frame. Regardless of whether this frame is spectacle-like or formed in a different shape suitable to adapt the wearable unit to the region of interest where it can be worn, preferably electronic circuits for connecting the one or more sensors with the data processing system are realized on said frame. In one very preferred exemplary embodiment, the data acquisition unit and/or a data analysis unit and/or a data interpretation unit or the entire data processing system may be printed on the frame. With regard to cost effective mass production, the electronic circuits may be realized on an external surface of the frame, preferably by being printed.

In the second aspect of the invention, the aforementioned object is accomplished by a kit-of-parts for the Human-Machine Interface according to the first aspect of claim 13. The kit-of-parts comprises a wearable unit, preferably a head-worn unit, at least two different hardware modules, such that the wearable unit is customizable for an individual user, wherein the at least two different hardware modules are connectable to the wearable unit, and wherein the at least two different hardware modules can be different types of sensors for collecting user data and/or different types of extensions of the wearable unit for positioning sensors for collecting user data, and optionally a data processing system for acquiring and processing said user data. The customizability and modularity of the invention can be realized in an advantageous way by providing a kit-of-parts that comprises at least two different hardware modules to be connected to the wearable unit, wherein the modules may advantageously be addible, removable, interchangeable, and/or exchangeable according to the preferences of an individual user.

In the third aspect of the invention, the aforementioned object is accomplished by a method for adjusting the Human-Machine Interface according to claim 14. The method for adjusting the HMI according to the first aspect comprises the steps of
recording data collected by the one or more sensors over time,
monitoring data patterns associated with respective identified individual user signals for a relevant change, and
upon detecting a relevant change in a data pattern associated with a respective individual user signal, taking one of the following adjustment measures according to the requirements of the individual user:
   a) adapting the sensitivity and/or configuration and/or localization of at least one of the one or more sensors;
   b) exchanging at least one of the at least two different hardware modules, preferably exchanging one of the one or more sensors;
   c) connecting at least one of the at least two different hardware modules, preferably adding an extension of the wearable unit for positioning at least one of the one or more sensors for acquiring data from an additional region of an individual user's head and/or body; and/or
   d) disconnecting at least one of the at least two different hardware modules.

The user signal may be a bio-signal of the individual user. By way of monitoring data patterns associated with respective identified individual user signals for a relevant change, such a change may point to a need to adjust the HMI, for example by changing the type, number and/or position of the hardware modules, in particular the sensors and extensions. For example, if the individual user is a patient suffering from motor neuron disorders, brain trauma or other neurological conditions causing impaired movement, such diseases may proceed over time. For example, while the patient may be perfectly able to use the HMI at a first point in time by moving one or more individual facial muscles and to send signals that generate EMG user data, this HMI setup will lose function as an individual user loses the ability to move individual facial muscles.

A relevant change in the respective data pattern in the EMG user data associated with the specific moves of the individual facial muscles may for example be a significant decrease in the signal strength, for example amplitude. This may point to a decrease in the intensity or strength of the individual facial muscle movements. Accordingly, the HMI may be adjusted, for example by increasing the EMG sensitivity - either on the software side or by exchanging the sensor for a more sensitive EMG electrode. Another possible reaction is to add or exchange at least one of the sensors/sensor types in order to collect different types of user data, for example from an additional region of an individual user's head and/or body.

The adjustment may also be such that one or more sensors are configured to be positioned automatically at an optimal location in a given region. To this end, sensors may be positioned in or near the given region such that they may search for the optimal location within a space that the sensor is able to reach. The automatic positioning of the sensor may be performed in a closed control loop, for example by processing user data representing the signal strength to acquire the best signal possible, and by using an embedded motor mechanism and/or any other type of mechanism suitable to move the sensor and/or the respective one or more extensions.

In the fourth aspect of the invention, the aforementioned object is accomplished by a method for adjusting the Human-Machine Interface according to claim 15. The method for adjusting the HMI according to the first aspect comprises the steps of
recording data collected by the one or more sensors over time,
acquiring additional signals from sensors, and
depending on the additional signal, taking one of the following adjustment measures according to the requirements of the individual user:
   a) adapting the sensitivity and/or configuration and/or localization of at least one of the one or more sensors;
   b) exchanging at least one of the at least two different hardware modules, preferably exchanging one of the one or more sensors;
   c) adding at least one of the at least two different hardware modules, preferably adding an extension of the wearable unit for positioning at least one of the one or more sensors for acquiring data from an additional region of an individual user's head and/or body; and/or
   d) disconnecting at least one of the at least two different hardware modules.

The occasion for adjustment of the HMI is not necessarily a relevant change in a data pattern associated with identified individual user signals. The occasion for adjustment of the HMI may also be any additional signal from sensors, including but not limited to the one or more sensors connectable to the wearable unit. The additional signal may include a signal of the individual user, for example an invasively and/or non-invasively collected bio-signal. Additional signals may also originate from an external sensor and may for example be a signal from the environment of the HMI, such as ambient light, temperature, air pressure, humidity and/or other types of environment signals.

### FIGURES

There are several ways how to design and further develop the teaching of the present invention in an advantageous way. To this end, it is to be referred to the patent claims subordinate to patent claim 1 on the one hand and to the following explanation of preferred examples of embodiments of the invention, illustrated by the drawing on the other hand. In connection with the explanation of the preferred embodiments of the invention by the aid of the drawing, generally preferred embodiments and further developments of the teaching will be explained. In the accompanying drawings
- Fig. 1: shows a schematic illustration of a first embodiment of the Human-Machine Interface according to the invention,
- Fig. 2: shows a schematic illustration of a second embodiment of the Human-Machine Interface according to the invention in a first configuration and
- Fig. 3: shows a schematic illustration of the second embodiment of the Human-Machine Interface according to the invention in a second configuration.

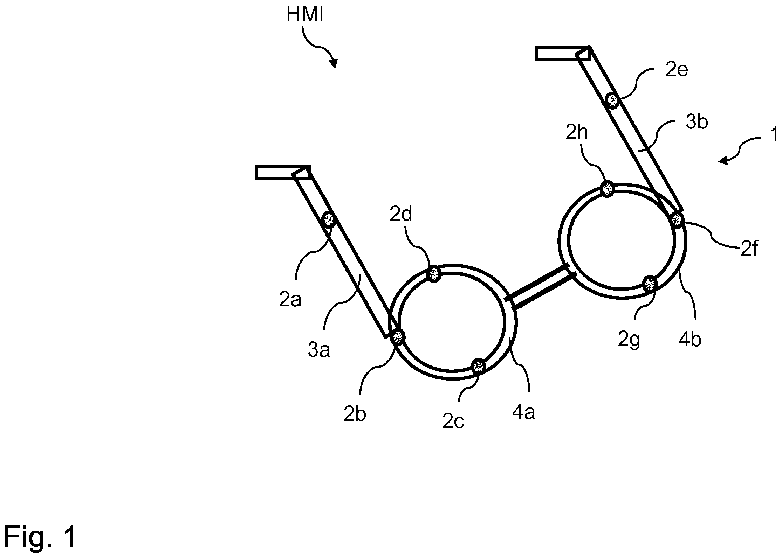

Fig. 1 shows a schematic illustration of a first embodiment of the Human-Machine Interface HMI according to the invention. The HMI comprises a wearable unit 1 realized as a head-worn unit with a spectacle-like frame. The HMI further comprises sensors 2a, 2b, 2c, 2d, 2e, 2f, 2g and 2h for collecting user data that are connected to the wearable unit 1, in particular the sensors 2a and 2e are connected to the temples 3a, 3b of the wearable unit's 1 spectacle like frame. The sensors 2b, 2c and 2d are connected to the rim 4a of the wearable unit's 1 spectacle like frame. The sensors 2f, 2g and 2h are connected to the rim 4b of the wearable unit's 1 spectacle like frame.

In this exemplary and schematic embodiment, the sensors 2a, 2b, 2c, 2d, 2e, 2f, 2g and 2h may all be realized hardware modules. The wearable unit is configured to be connected to at least two different sensors - or hardware modules - such that the wearable unit is customizable for an individual user.

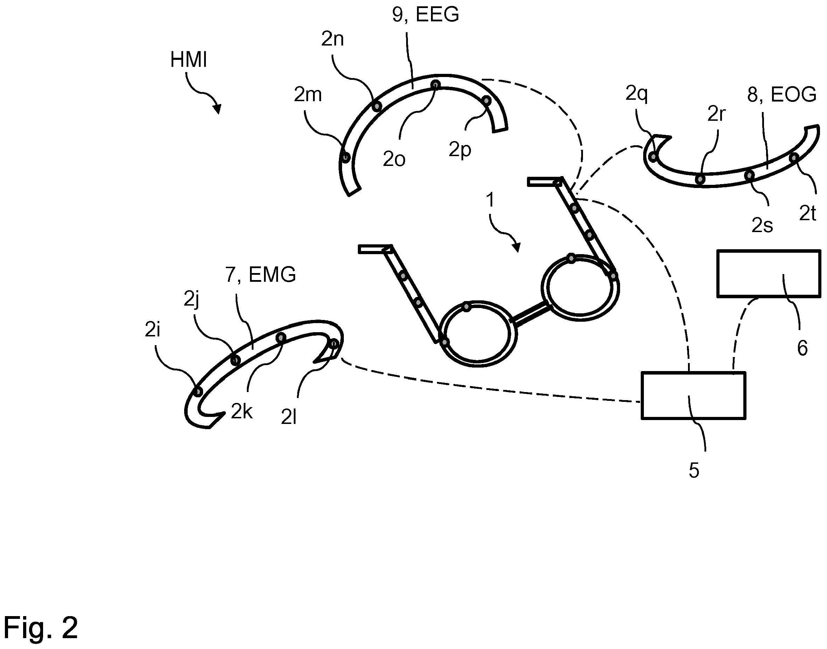

Fig. 2 shows a schematic illustration of a second embodiment of the Human-Machine Interface HMI according to the invention in a first configuration. The wearable unit 1 realized as a head-worn unit with a spectacle-like frame similar to the first embodiment of Fig. 1. In Fig. 2, a data processing system 5 and additional hardware 6 is illustrated. The data processing system 5 may be a mobile device and include a data acquisition unit, a data analysis unit and a data interpretation unit integrated in a single hardware device. The additional hardware 6 may be a speaker system externally to the head one unit 1.

Additionally, three extensions 7, 8 and 9 are depicted in Fig. 2. Extension 7 is wirelessly connected to the data processing system 5, which is illustrated by a dashed line.

Extension 7 comprises sensors 2i, 2j, 2k and 2l, which are electrodes for collecting electromyography (EMG) user data. Extension 7 is formed in the exemplary shape of the bracket and can be worn/positioned on or close to a region of interest of an individual user's body and/or head.

Extension 8 is wirelessly connected to the wearable unit 1, which is illustrated by a dashed line. Extension 8 comprises 2m, 2n, 2o and 2p, which are electrodes for collecting electrooculography (EOG) user data. Extension 8 is formed in the exemplary shape of the bracket and can be worn/positioned on or close to an individual user's eye.

Extension 9 is wirelessly connected to the wearable unit 1, which is illustrated by a dashed line. Extension 9 comprises 2q, 2r, 2s and 2t, which are electrodes for collecting electroencephalography (EEG) user data. Extension 9 is formed in the exemplary shape of the bracket and can be worn/positioned on or close to a region of interest, for example on the back of an individual user's head.

Each extension 7, 8, 9 can have different types of sensors. The same sensors 2i, 2j, 2k, 2l, 2m, 2n, 2o, 2p, 2q, 2r, 2s and 2t can acquire EEG, EOG or EMG depending on their location as well as on the respective signal processing method. In other words, EEG, EMG and EOG user data can be collected with the same sensors. Moreover, there may be more than two extensions, for example three or four extensions, preferably 5 to 10, 11 to 15, 16 to 20, 21 to 25 or more than 25 extensions of the wearable unit.

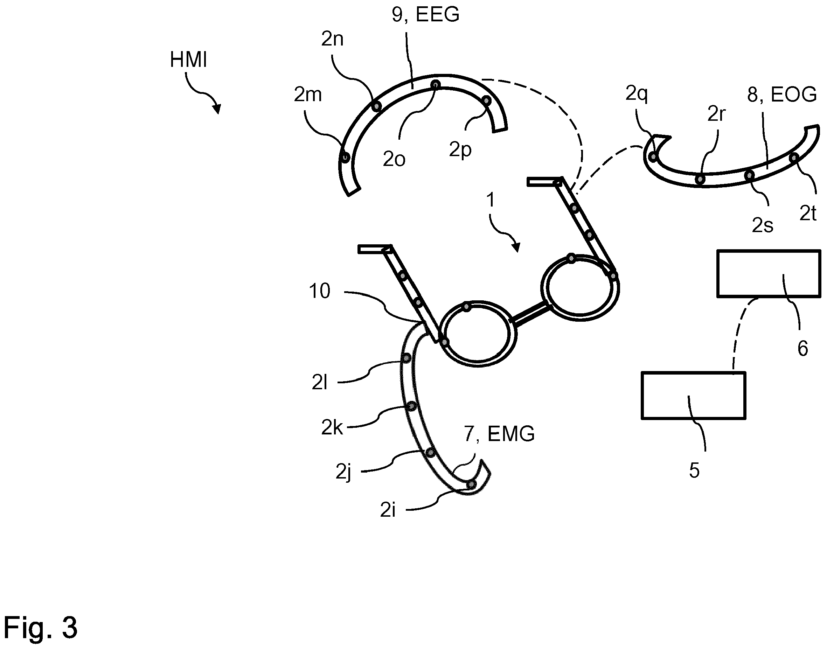

Fig. 3 shows a schematic illustration of the second embodiment of the Human-Machine Interface according to the invention in a second configuration. Different from the first configuration depicted in Fig. 2, the extension 7 in Fig. 3 is connected physically to the head word unit 1 at interface 10, which may be a port, connectors or plug.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

### List of reference signs

| | |
|---|---|
| 1 | Wearable unit |
| 2a, 2b, 2c, 2d, 2e, 2f, | |
| 2g, 2h,2i, 2j, 2k, 2l, 2m, | |
| 2n, 2o, 2p, 2q, 2r, 2s, 2t | Sensors |
| 3a, 3b | Temples |
| 4a, 4b | Rims |
| 5 | Data processing system |
| 6 | Hardware |
| 7 | Extension |
| 8 | Extension |
| 9 | Extension |
| 10 | Interface |
| HMI | Human-Machine-interface |
| EEG | Electroencephalography |
| EMG | Electromyography |
| EOG | Electrooculography |

## Claims

1. A Human-Machine Interface (HMI), comprising
a wearable unit (1), preferably a head-worn unit,
one or more sensors (2a, 2b, 2c, 2d, 2e, 2f, 2g, 2h, 2i, 2j, 2k, 2l, 2m, 2n, 2o, 2p, 2q, 2r, 2s, 2t) for collecting user data, wherein at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t) is connectable to the wearable unit (1), and
a data processing system (5) for acquiring and processing said user data,
**characterized in that**
the wearable unit (1) is configured to be connected to at least two different hardware modules, such that the wearable unit is customizable for an individual user.

2. The Human-Machine Interface (HMI) of claim 1,
wherein one of the one or more sensors (2a, 2b, 2c, 2d, 2e, 2f, 2g, 2h, 2i, 2j, 2k, 2l, 2m, 2n, 2o, 2p, 2q, 2r, 2s, 2t) is realized as one of the at least two different hardware modules.

3. The Human-Machine Interface (HMI) of claim 1 or claim 2,
wherein the at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t) includes an electrode for collecting electrooculography (EOG), electromyography (EMG) and/or electroencephalography (EEG) user data, wherein preferably
the data processing system (5) is configured to process user data from at least two modalities comprising electrooculography (EOG), electromyography (EMG) and electroencephalography (EEG) in order to interpret an individual user's signal.

4. The Human-Machine Interface (HMI) of any one of the preceding claims,
wherein the at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t) include at least two different kinds of sensors for collecting time series signals and/or frequency series signals, preferably including an invasively and/or non-invasively collected signal and/or a blood oxygenation level-dependent signal.

5. The Human-Machine Interface (HMI) of any one of the preceding claims,
wherein the data processing system (5) is configured to provide the individual user with information, wherein said information is provided in a format perceivable by the individual user, preferably in a non-visual format.

6. The Human-Machine Interface (HMI) of any one of the preceding claims,
wherein the wearable unit comprises speaker and/or display devices.

7. The Human-Machine Interface (HMI) of any one of the preceding claims,
wherein the data processing system (5) comprises a data acquisition unit and/or a data analysis unit and/or a data interpretation unit.

8. The Human-Machine Interface (HMI) of claim 7,
wherein the data acquisition unit and/or the data analysis unit and/or the data interpretation unit are realized in separate hardware devices.

9. The Human-Machine Interface (HMI) of claim 7,
wherein the data acquisition unit and/or the data analysis unit and/or the data interpretation unit are integrated into a single hardware device.

10. The Human-Machine Interface (HMI) of any one of the preceding claims,
wherein one of the at least two different hardware modules is implemented as an extension (7, 8, 9) of the wearable unit (1),
wherein the extension (7, 8, 9) comprises at least one of the one or more sensors (2i, 2j, 2k, 2l, 2m, 2n, 2o, 2p, 2q, 2r, 2s, 2t) for collecting user data from at least one additional region of the individual user's head and/or body.

11. The Human-Machine Interface (HMI) of any one of the preceding claims,
wherein the wearable unit (1) comprises a spectacle-like frame, preferably including rims (4a, 4b) , hinges and/or temples (3a, 3b).

12. The Human-Machine Interface (HMI) of any one of the preceding claims,
wherein the wearable unit (1) comprises a frame, preferably wherein electronic circuits for connecting the one or more sensors with the data processing system are realized on said frame.

13. Kit-of-parts for the Human-Machine Interface (HMI) as defined in any one of the preceding claims, comprising
a wearable unit (1), preferably a head-worn unit,
at least two different hardware modules, such that the wearable unit is customizable for an individual user, wherein the at least two different hardware modules are connectable to the wearable unit (1), and wherein the at least two different hardware modules can be different types of sensors (2a, 2b, 2c, 2d, 2e, 2f, 2g, 2h, 2i, 2j, 2k, 2l, 2m, 2n, 2o, 2p, 2q, 2r, 2s, 2t) for collecting user data and/or different types of extensions of the wearable unit for positioning sensors for collecting user data, and optionally
a data processing system (5) for acquiring and processing said user data.

14. A method for adjusting the Human-Machine Interface (HMI) of any one of the claims 1 to 12, comprising the steps of
recording data collected by the one or more sensors (2a, 2b, 2c, 2d, 2e, 2f, 2g, 2h, 2i, 2j, 2k, 2l, 2m, 2n, 2o, 2p, 2q, 2r, 2s, 2t) over time,
monitoring data patterns associated with respective identified individual user signals for a relevant change, and
upon detecting a relevant change in a data pattern associated with a respective individual user signal, taking one of the following adjustment measures according to the requirements of the individual user:
a) adapting the sensitivity and/or configuration and/or localization of at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t);
b) exchanging at least one of the at least two different hardware modules, preferably exchanging one of the one or more sensors (2a, 2b, 2c, 2d, 2e, 2f, 2g, 2h, 2i, 2j, 2k, 2l, 2m, 2n, 2o, 2p, 2q, 2r, 2s, 2t);
c) connecting at least one of the at least two different hardware modules, preferably adding an extension (7, 8, 9) of the wearable unit (1) for positioning at least one of the one or more sensors (2i, 2j, 2k, 2l, 2m, 2n, 2o, 2p, 2q, 2r, 2s, 2t) for acquiring data from an additional region of an individual user's head and/or body; and/or
d) disconnecting at least one of the at least two different hardware modules.

15. A method for adjusting the Human-Machine Interface of any one of the claims 1 to 12, comprising the steps of
recording data collected by the one or more sensors (2a, 2b, 2c, 2d, 2e, 2f, 2g, 2h, 2i, 2j, 2k, 2l, 2m, 2n, 2o, 2p, 2q, 2r, 2s, 2t) over time,
acquiring additional signals from sensors, and
depending on the additional signal, taking one of the following adjustment measures according to the requirements of the individual user:
a) adapting the sensitivity and/or configuration and/or localization of at least one of the one or more sensors (2a, 2b, 2c, 2d, 2e, 2f, 2g, 2h, 2i, 2j, 2k, 2l, 2m, 2n, 2o, 2p, 2q, 2r, 2s, 2t);
b) exchanging at least one of the at least two different hardware modules, preferably exchanging one of the one or more sensors (2a, 2b, 2c, 2d, 2e, 2f, 2g, 2h, 2i, 2j, 2k, 2l, 2m, 2n, 2o, 2p, 2q, 2r, 2s, 2t);
c) connecting at least one of the at least two different hardware modules, preferably adding an extension (7, 8, 9) of the wearable unit (1) for positioning at least one of the one or more sensors (2i, 2j, 2k, 2l, 2m, 2n, 2o, 2p, 2q, 2r, 2s, 2t) for acquiring data from an additional region of an individual user's head and/or body; and/or
d) disconnecting at least one of the at least two different hardware modules.
